# EUROPEAN PATENT SPECIFICATION

(11) **EP 3 975 883 B1**
(45) Date of publication and mention of the grant of the patent: **06.08.2025**
(21) Application number: 20813662.2
(22) Date of filing: 27.05.2020
(51) Int. Cl.: A61B 17/17, A61B 1/06, A61B 17/00, A61B 17/34, A61B 17/56, A61B 17/60, A61F 2/08, A61B 34/20, A61B 90/10

(54) **ADAPTER FOR A SURGICAL ACCESS DEVICE**
ADAPTER FÜR EINE CHIRURGISCHE ZUGANGSVORRICHTUNG
ADAPTATEUR POUR UN DISPOSITIF D'ACCÈS CHIRURGICAL

(30) Priority: 31.05.2019 US 201962855409 P
(43) Date of publication of application: 06.04.2022
(73) Proprietor: Stryker Corporation, Portage, MI 49002 (US)
(72) Inventor: MARK, Joseph L., Indianapolis, IN 46260 (US); DOUGHERTY, Brian C., Terre Haute, IN 47803 (US); CARR, Jacob J., Indianapolis, IN 46220 (US)
(74) Representative: Schott, Jakob Valentin
(86) International application number: PCT/US2020/034671
(87) International publication number: WO 2020/243156

(56) References cited:
- EP-A2- 1 949 860
- WO-A1-2015/175635
- WO-A2-2015/134562
- US-A- 4 654 030
- US-A- 5 662 613
- US-A1- 2002 010 474
- US-A1- 2008 039 839
- US-A1- 2009 306 675
- US-A1- 2010 022 844
- US-A1- 2013 184 730
- US-A1- 2014 358 129
- US-A1- 2016 135 800
- US-A1- 2016 199 575
- US-A1- 2018 125 603
- US-A1- 2018 153 564
- US-A1- 2019 117 254
- US-B1- 6 241 729

## Description

### FIELD

The present disclosure relates generally to adapters for use with surgical access devices.

### BACKGROUND

This section provides background information related to the present disclosure and is not necessarily prior art. The documents US 2018 / 125 603 A1, WO 2015 / 175 635 A1, US 2016 / 135 800 A1, US 5 662 613 A, EP 1 949 860 A2 and WO 2015 134 562 A2 were cited. Particularly, EP 1 949 860 A2 discloses a system with an obturator and a working tube; wherein the obturator includes an upper control housing and a pair of spreading legs.

Diagnosis and treatment of conditions affecting the brain are among the most difficult and complex problems that face the medical profession. The brain is a complex and delicate soft multi-component tissue structure that controls bodily functions through a complex neural network connected to the rest of the body through the spinal cord. The brain and spinal cord are contained within and protected by significant bony structures, e.g., the skull and the spine. Given the difficulty of accessing the brain through the hard bony protective skull and the delicate network and complex interactions that form the neural communication network contained within the brain that define the human body's ability to carry on its functions of speech, sight, hearing, functional mobility, reasoning, emotions, respiration and other metabolic functions, the diagnosis and treatment of brain disorders presents unique challenges not encountered elsewhere in the body.

For example, abnormalities such as intracranial cerebral hematomas (ICH), abscesses, Glioblastomas (GB) and metastases (mets) manifest themselves in the intraparenchymal subcortical space (i.e., the white matter) of the brain are particularly challenging to access, let alone treat. The white matter and cortex of the brain contain eloquent communication structures (neural network) which are located in the cortical and subcortical space, called fiber tracts and fascicles. Thus, traditionally, unless the ICH, GB, and/or mets where considered anything but "superficial," such conditions have been considered inoperable, simply because getting to the abnormality ICH, GB and/or mets are considered just as damaging as letting the condition take its course. Similarly, tissue abnormalities such as tumors, cysts and fibrous membrane growths which manifest within the intraventricular space of the brain are considered challenging to safely access and often inoperable, due to their locations within the brain.

To assist in diagnosis and subsequent treatment of brain disorders, clear, accurate imaging of brain tissue through the skull is required. In recent years significant advances have been made in imaging technology, including stereotactic X-ray imaging, Computerized Axial Tomography (CAT), Computerized Tomographic Angiography (CTA), Position Emission Tomography (PET) and Magnetic Resonance Imaging (MRI), Diffusion Tensor Imaging (DTI) and Navigation systems (instrument position tracking systems). These imaging devices and techniques permit the surgeon to observe conditions within the brain in a non-invasive manner without opening the skull, as well as provide a map of critical structures surrounding an area of interest, including structures such as blood vessels, membranes, tumor margins, cranial nerves, including fiber tracts and fascicles. If an abnormality is identified through the use of one or more imaging modalities and/or techniques, it may be necessary or desirable to surgically intervene.

Once a course of action has been determined based upon one or more imaging techniques, a surgical treatment may be necessary or desired. To operate surgically within the brain, access must be obtained through the skull and delicate and eloquent brain tissue such as blood vessels, the lymphatic system and nerves overlying and surrounding the abnormality that can be adversely affected by slight disturbances and disruptions. Therefore, great care must be taken in operating on the brain so as not to disturb these eloquent tissues to prevent adverse consequences resulting from an intervention.

Traditionally, accessing abnormalities which manifest in deeper spaces within the brain has meant a need for a surgery that creates a disruptive invasive approach. In some instances, in order to obtain access to target tissue, a substantial portion of the skull is removed and entire sections of the overlying brain are retracted to obtain access. For example, surgical brain retractors are used to pull apart or spread delicate brain tissue, which can produce transient and/or permanent deficits. In some instances, a complication known as "retraction injury" may occur due to use of brain retractors. Of course, such techniques are not appropriate for all situations, and not all patients are able to tolerate and recover from such invasive techniques.

It is also known to access certain portions of the brain by creating a burr hole craniotomy, but only limited surgical techniques may be performed through such smaller openings. In addition, some techniques have been developed to enter through the nasal passages, opening an access hole through the bone to remove skull-based tumors, for example, in the area of the pituitary. These approaches are referred to as Expanded Endonasal Approaches (EEA).

A significant advance in brain surgery is stereotactic surgery involving a stereotactic frame correlated to stereotactic X-ray images and MRI to guide a navigational system probe or other surgical instrument through an opening formed in the skull through brain tissue to a target lesion or other body. A related advance is frameless image guidance, in which an image of the surgical instrument is superimposed on a pre-operative image to demonstrate the location of the instrument to the surgeon and trajectory of further movement of the probe or instrument.

It is known to access certain portions of the brain with surgical access systems. An example of a surgical access systems used to access certain portions of the brain may be found in US 2016 / 0 128 722 A1. A navigation member may be used with an obturator by slidingly engaging with the obturator.

One issue with surgical access systems that arises is compatibility a variety of known navigational arrangements and systems. For example, some navigational system probes may be incompatible because they are sized differently, and therefore unable to engage with an existing obturator directly. More specifically, certain navigational system probes may be too wide and therefore unable to be seated within an obturator without alterations being made to the obturator. Alternatively, a navigational system probe may be too narrow and may not be able to be properly seated within an obturator. Additionally, incompatibility may occur due to the material from which the obturator is constructed which can interfere or block the signals produced by the navigational systems, especially those navigational system that utilize electromagnetic signals (an example of which is the AxiEM System manufactured by Medtronic). Notwithstanding the foregoing advances in surgical navigation, there remains a need for improved surgical techniques and apparatuses for operating on brain tissue, including providing multi-compatible navigational systems.

### SUMMARY

This section provides a general summary of the disclosure, and is not a comprehensive disclosure of its full scope or all of its features. The invention is defined by independent claim 1, with further embodiments defined by the dependent claims. Implementations of the disclosure may include one or more of the following optional features.

One aspect of the disclosure provides an adapter for use with a surgical access assembly. The adapter comprises an adapter body and a positioning member. The adapter body is defined by a distal end and a proximal end opposite the distal end. The positioning member extends from the distal end of the adapter body. A channel extends through the adapter body, extending from the proximal end and toward the distal end.

In one exemplary arrangement, the adapter body further comprises a locking mechanism to secure a navigation element to the adapter against movement.

In one exemplary arrangement, the channel terminates in a closed distal end that defines a seating portion. The distal end tapers inwardly to define the seating portion. A window may be provided to provide visual access to the channel, adjacent the seating portion.

In one exemplary arrangement, the adapter body further includes a grip portion disposed adjacent to the proximal end of the adapter body.

In one exemplary arrangement, the adapter body includes an engagement section that includes an annular angled surface encircling the proximal end of the positioning member.

Another aspect of the disclosure provides an adapter for a surgical access assembly comprising a first adapter casing, a second adapter casing and a mounting piece. The first and second adapter casings are selectively engageable with one another. The first and second adapter casings cooperate to define a channel that is configured to receive a navigation element. The mounting piece is selectively engaged with the first and second adapter casings.

In one exemplary arrangement, the first adapter casing includes a first navigation groove defined between a proximal end of the first adapter casing and a distal end of the first adapter casing, and the second adapter casing includes a second navigation groove defined between a proximal end of the second adapter casing and a distal end of the second adapter casing, the first and second navigation grooves collectively define the channel.

In one exemplary arrangement, a portion of the mounting piece is disposed within a portion of an inner surface of the first adapter casing and a portion of an inner surface of the second adapter casing to secure the mounting piece to the first and second adapter casings.

In one exemplary arrangement, the first adapter casing includes an engagement channel adjacent to the distal end of the first adapter casing, the second adapter casing includes an engagement channel adjacent to the distal end of the second adapter casing, and the mounting piece includes a securing portion that engages with the engagement channel of the first adapter casing and the engagement channel of the second adapter casing.

In one exemplary arrangement, an engagement surface of the first adapter casing defines one or more first cooperating members, and wherein an engagement surface of the second adapter casing includes one or more second cooperating members configured to engage with the one or more first cooperating members of the inner surface of the first adapter casing to connect the first and second adapter casings together.

In one exemplary arrangement, the mounting piece further includes a window. The window may further include a spring element that is biased inwardly toward the channel.

In one exemplary arrangement, the first and second adapter casings are hingedly connected to the mounting portion.

Further areas of applicability will become apparent from the description provided herein. The description and specific examples in this summary are intended for purposes of illustration only and are not intended to limit the scope of the present disclosure.

### BRIEF DESCRIPTIO OF THE DRAWINGS

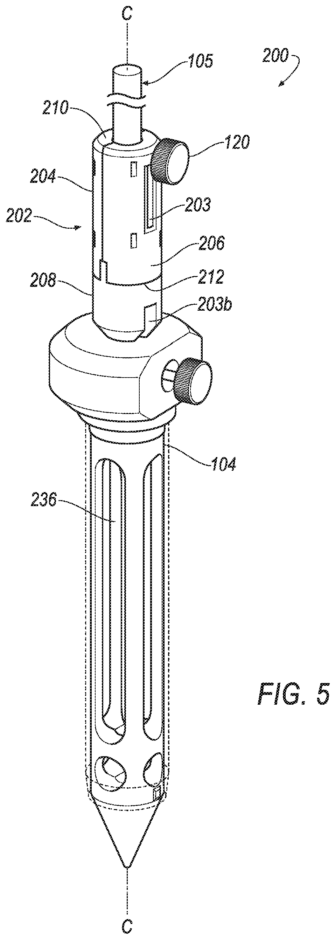

The drawings described herein are for illustrative purposes only of selected configurations and not all possible implementations, and are not intended to limit the scope of the present disclosure. FIGS. 5-7 show embodiments according to the invention. The rest of the figures are not according to the invention but are considered useful for its understanding.
FIG. 1 A is a perspective view of a portion of an exemplary surgical access assembly assembled with a navigation adapter.
FIG. IB is a plan view of an obturator for use with an adapter.
FIG. 2A is a front elevational view of the adapter of FIG. 1.
FIG. 2B is a side elevational view of the adapter of FIG. 1.
FIG. 3 A is a cross-section view of the adapter of FIG. 1 taken along lines 3A-3A of FIG. 2B, as well as an exploded view of an exemplary navigational element.
FIG. 3B is an enlarged view of area 3B taken from FIG. 3 A.
FIG. 4A is a top plan view the adapter of FIG. 1.
FIG. 4B is a bottom plan view of the adapter of FIG. 1.
FIG. 5 is a perspective view of an exemplary surgical access assembly assembled with an exemplary navigation adapter.
FIG. 6 is a cross-section view of the adapter of FIG. 5 taken along a center line extending through the adapter.
FIG. 7 is an exploded view of the adapter of FIG. 5.
FIG. 8 is a partially exploded cross-sectional view of a further exemplary navigation adapter and navigational element.
FIG. 9 is a side elevational view of the adapter of FIG. 8.
FIG. 10 is a front cross-sectional view of the adapter and navigational element of FIG. 8, assembled.
FIG. 11 is a front cross-sectional view of the adapter and navigational element of FIG. 8 assembled to an obturator.
FIG. 12 is an enlarged area 12 taken from area 12 in FIG. 11.
FIG. 13 is a partial front cross-sectional view of the adapter and navigational element assembled together.
FIG. 14 is a front elevational view of the adapter and navigational element of FIG. 12 assembled to an obturator.
FIG. 15 is a perspective view of a further exemplary arrangement of a navigational adapter.

Corresponding reference numerals indicate corresponding parts throughout the drawings.

### DETAILED DESCRIPTION

Referring now to the discussion that follows and also to the drawings, illustrative approaches to the disclosed assemblies are shown in detail. Although the drawings represent some possible approaches, the drawings are not necessarily to scale and certain features may be exaggerated, removed, or partially sectioned to better illustrate and explain the present disclosure. Further, the descriptions set forth herein are not intended to be exhaustive or otherwise limit or restrict the claims to the precise forms and configurations shown in the drawings and disclosed in the following detailed description.

Described herein is an adapter for a surgical access assembly and various components for use in same. The components disclosed herein provide surgeons with an enhanced ability to minimize trauma to the patient, while providing efficient improved minimally invasive surgical techniques, such as, for example, during intracranial surgical techniques.

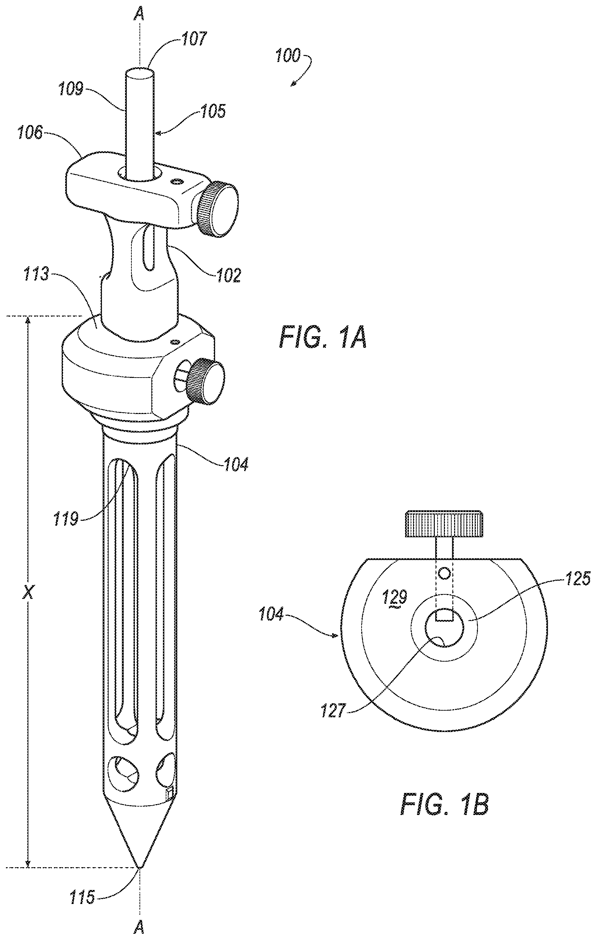

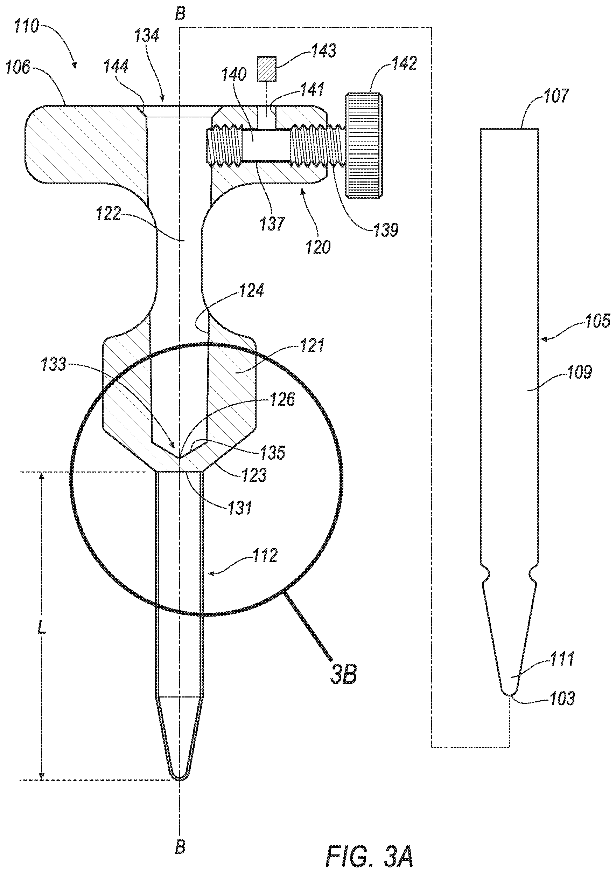

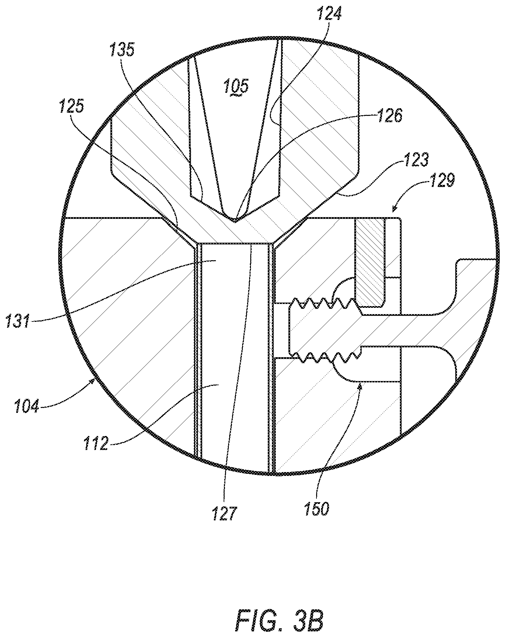

Referring to FIG. 1 A, a perspective view of a medical access assembly 100 including a navigation adapter 102 mounted to an obturator 104 of a surgical access system is shown. A navigational element 105 is selectively engageable with adapter 102 to be used with obturator 104 of a surgical access system. In use, the obturator 104 is selectively slidably engaged with an outer sheath (not shown) to provide access to an area of interest in a patient. An example of an exemplary surgical access system may be found in US 2016 / 0 128 722 A1. In one exemplary arrangement, the navigational element 105 (best seen in FIG. 3) is defined by a proximal end 107, a body portion 109 and a distal end 111. When navigational element 105 is positioned within adapter 102, the body portion 109 will extend along an axis A- A and beyond an adapter proximal end 106. By having the proximal end 107 extend proximally from the adapter proximal end 106, the navigational element 105 is able to selectively connect to a navigation system (not shown). In some implementations, navigational element 105 is a navigation probe having a tapered distal tip 103. In addition, navigational element 105 may include an annular notch 101, which may receive a sealing element such as an O-ring (not shown).

When navigational element 105 is assembled with adapter 104, body portion 109 extends about axis A-A. As will be discussed in greater detail below, adapter 102 enables navigational element 105 to be used with obturator 104, even if navigational element 105 is incompatible or unable to operationally engage with obturator 104. As will be explained in further detail below, an offset X between a proximal end face 113 and a distal tip 115 of the obturator 104 is a known parameter. As the adapter 102 is configured to locate the distal end 111 of the navigational element 105 in the same plane as the proximal end face 113 of the obturator 105, a navigation system can calculate the location of the distal tip 115 of the obturator 105 by factoring in the known offset X with the location of the distal end 111.

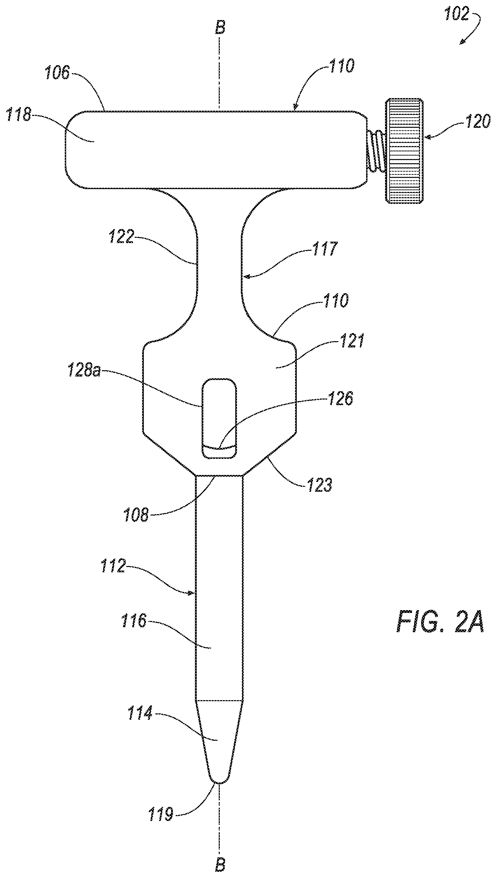

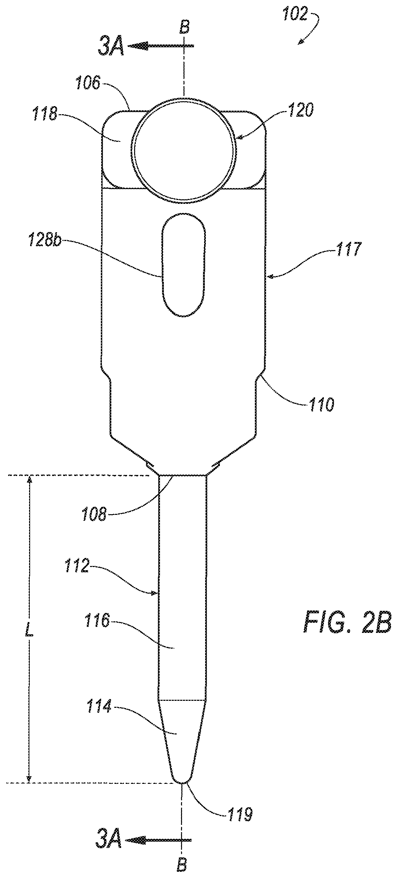

Referring now to FIGS. 2A-2B, details of the adapter 102 will be described in further detail. Adapter 102 is defined by an adapter body 117 and a positioning member 112. The adapter body 117 is further defined by proximal end 106 and distal end 108. In one exemplary arrangement, a handle portion 110 is disposed at the proximal end 106. The positioning member 112 extends from the distal end 108 of the adapter body 117 and generally along an axis B-B. The positioning member 112 is defined by a length L (FIG. 2B) and further includes a tip member 114. In one exemplary arrangement, the tip member 114 is configured to narrow from a body section 116 of the positioning member 112 to an adapter distal tip 119. Other tip member 114 configurations are also contemplated such as a rounded distal tip. In some implementations, the adapter body 117 and positioning member 112 are molded together as a unitary body. In other implementations, adapter body 117 and positioning member 112 are in selective engagement with each other. Handle portion 110 may be further defined by grip portions 118 that extends outwardly from axis B-B. In one exemplary arrangement, grip portion 118 is configured to be generally perpendicular to axis B-B. In some implementations, Adapter 102 may further include a locking mechanism 120, which will be described in more detail below. Though FIGS. 2A-2B depict locking mechanism 120 disposed on grip portion 118, it should be noted that locking mechanism 120 may be disposed anywhere on handle portion 110.

As best seen in FIG. 2 A, adapter body 117 further includes an engagement section 121 disposed distal to handle portion 110. As best seen in FIGS. 3A and 3B, engagement section 121 includes an annular angled surface 123 that encircles a proximal end 131 of positioning member 112. Angled surface 123 is configured to frictionally engage an annular chamfer 125 disposed about a proximal opening 127 formed in a top surface 129 of obturator 104 (see FIGS. 1 A and 3B). Angled surface 123 is angled inwardly toward a central axis of the positioning member 112

The handle portion 110 may include an arcuate section 122 disposed between proximal end 106 and engagement section 121. Arcuate portion 122 may advantageously allow a physician to comfortably hold adapter 102, reducing physician fatigue and making adapter 102 easier to manipulate in the surgical field.

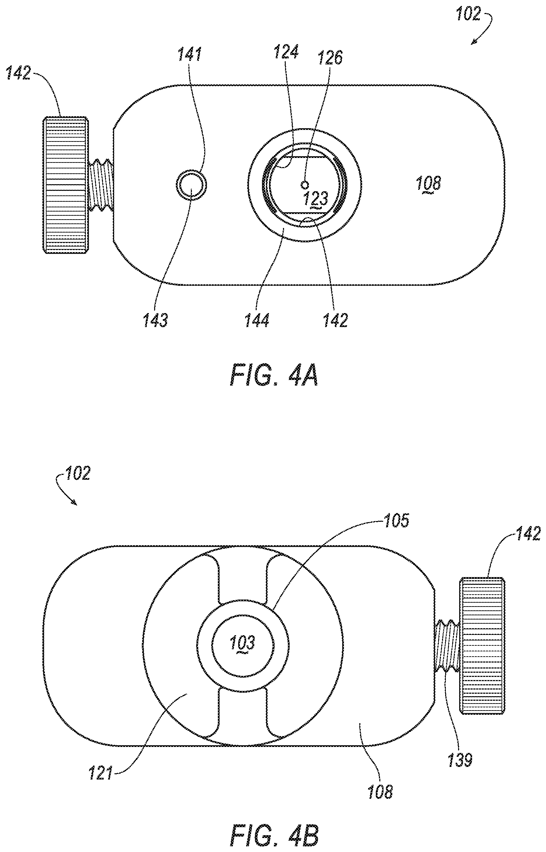

With continued reference to FIGS. 2-4, handle portion 110 includes an axial channel 124. Channel 124 extends from an opening 134 disposed at proximal end 106, along axis B-B, but terminates proximally of the positioning member 112. An inwardly extending chamfer 144 may be disposed around opening 134 to assist in directing navigational element 105 within channel 124. Channel 124 is sized to receive navigational element 105 therewithin. It is contemplated that various adapters 102 may be provided with channels 124 being sized with predetermined diameters that permit known sized navigational elements 105 to be inserted therein. Alternatively, for those arrangements where the diameter of the channel 124 is significantly larger than the diameter of the navigational element 105, an optional sizing sleeve (not shown) may be provided that has an outside diameter that is receivable within the channel 124, and has an inside diameter that receives the navigational element 105 snugly. As a further option, navigational member 105 may be provided with the annular notch 101 formed on an outside surface of the body portion 109, the annular notch 101 receiving an o-ring or a spring clip therein to frictionally retain the navigational element 105 within the channel 124.

Disposed at a distal end 133 of channel 124 is a seating portion 126. In one exemplary arrangement, seating portion 126 is defined by an annular inwardly directing surface 135. The inwardly directing surface 135 serves to self-direct and center navigational element 105 when inserted within adapter 102. Other configurations of seating portion 126 are also contemplated, such as tongue and groove arrangements.

Handle portion 110 may also define at least one window 128. Window 128 allows a user to visually confirm the placement of navigational element 105 (FIG. 1) when disposed within adapter 102. Windows 128 also serve to reduce the weight of adapter 102, thereby making adapter 102 easier to manipulate in the surgical field. Reducing the weight of adapter 102 also reduces physician fatigue during a surgical procedure.

In the exemplary arrangement, at least one window 128a may be disposed adjacent to positioning member 112, enabling a user to visually confirm that a navigational element 105 is in proper contact with seating portion 126. As depicted in FIG. 2A, window 128a may be a front- facing window, such that the "front" of the adapter 102 is, with respect to the width of grip portion 118, being at its widest. Additionally or alternatively, in an exemplary arrangement, a window 128b may be positioned adjacent to grip portion 118 to visually confirm that the navigational element 105 is disposed along the axis B-B. As depicted in FIG. 2B, window 128b may be a side-facing window, such that the "side" of the adapter is with respect to the width of grip portion 118 being at its narrowest. Adapter 102 having both a front-facing window 128a and a side-facing window 128b may be advantageous in allowing a user to ensure proper placement of navigational element 105 (FIG. 1) by viewing navigational element 105 from two different angles. In some implementations, at least a portion of adapter 102 may be made of a transparent material, which also allows a user to visually confirm the placement of navigational element 105 when inside adapter 102.

Referring to FIG. 3A-4B, in some implementations, locking mechanism 120 of adapter 102 may operatively fix navigational element 105 (FIG. 3) to adapter 102. More specifically, in one exemplary arrangement, grip portion 118 (or other portion of adapter 102) is provided with a receiving aperture 137 that is configured to receive a locking member 139. In one exemplary arrangement, receiving aperture 137 is threaded. Receiving aperture 137 is in communication with channel 124. A retaining channel 141 extends generally perpendicular to the receiving aperture 137. The locking member 139 includes a reduced diameter section 140. After locking member 139 is at least partially engaged within receiving aperture 137, a retaining member 143 is positioned within retaining channel 141 so as to be disposed adjacent to the reduced diameter section 140. In operation, once the retaining member 143 is inserted within the retaining aperture 137, an actuating member 142 is rotated to move the retaining member 143 toward the channel 124 and into engagement with the navigational element 105, thereby locking the navigational element 105 within the adapter 102. The retaining member 143 prevents locking mechanism 120 from being completely disengaged from adapter 102 and entering the surgical field and, particularly, it prevents locking mechanism 120 from contacting exposed body tissue in the surgical field.

As shown in FIG. 3B, obturator 104 may also be provided with a locking mechanism 150 that is similar to locking mechanism 120.

In operation, the navigational element 105 is inserted through opening 134 and into channel 124 until a distal tip 103 engages seating portion 126. A user may view the navigational element 105 while in the actuator 102 by looking through windows 128. Once proper positioning is confirmed, if available, locking mechanism 120 is activated to secure navigational element 105 in place within the actuator 102.

Once navigational element 105 is secured to the actuator 102, the adapter 102 is joined to the obturator 104. More specifically, the positioning member 112 is inserted within the proximal opening 127 of the obturator 104. As the positioning member 112 is elongated, a portion of the positioning member 112 will be disposed within the obturator 104, extending proximally from the top surface of the obturator 104, as shown in FIG. 1. The annular angled surface 123 of the engagement section 121 engages the annular chamfer 125 in a complementary manner. Once positioned, locking mechanism 150 may be actuated to lock the positioning member 112 to the obturator 104. As discussed above, once so assembled, the seating portion 126 serves to position the distal tip 103 of the navigational element 105 in alignment with the top surface 129 of the obturator 104. The proximal end 107 of the navigational element 105 is secured to the navigational system. Thus, when the obturator 104 is assembled to the outer sheath of a surgical access assembly, because the offset X between the top surface 129 and the distal tip 115 of the obturator 104 is a known predefined length, the location of the distal tip 115 may be determined while the surgical access assembly is in use, as the distal tip 103 of the navigational element 105 will be known by correlating the offset X with the location of the distal tip 103 of the navigational element 105.

Referring now to FIGS. 5-7, a perspective view of a medical access assembly 200 including an alternative adapter 202 mounted to an obturator 104 of a surgical access assembly is shown. Similar to adapter 102, adapter 202 is configured to selectively engage with obturator 104 of a surgical access system to allow different sized navigational elements 105 to be used with obturator 104. According to the invention, adapter 202 comprises a first adapter casing 204, a second adapter casing 206, and a mounting piece 208. First and second adapter casings 204, 206 are each defined by a proximal end 210 and a distal end 212. In some implementations, at least one of first adapter casing 204, second adapter casing 206, and mounting piece 208 may include locking mechanism 120 (best seen in FIG. 5).

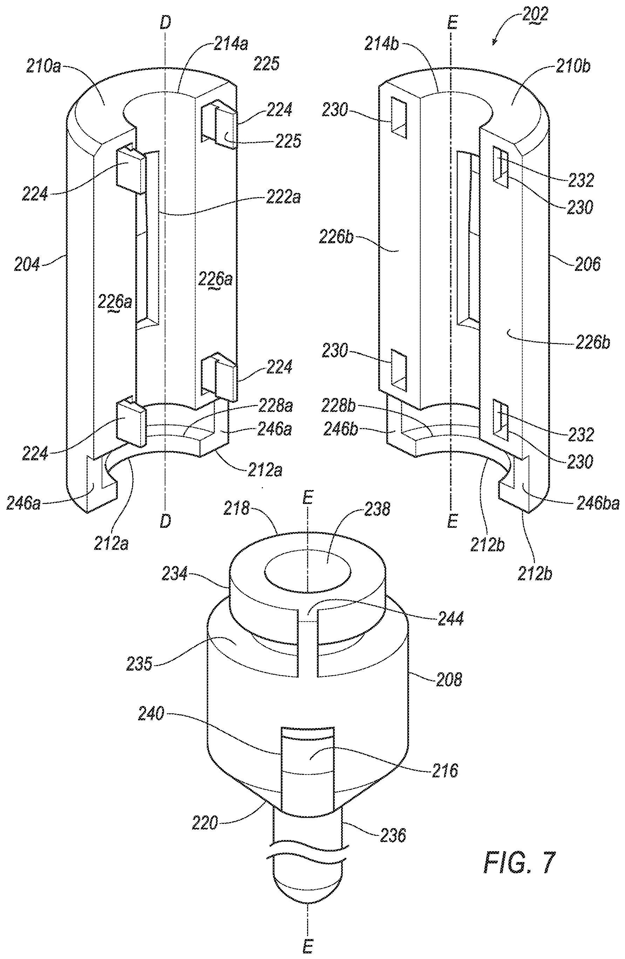

Referring now to FIG. 7, first adapter casing 204 extends partially about an axis D-D and is defined by a proximal end 210a and a distal end 212a. In one exemplary arrangement, first adapter casing 204 has a generally U-shaped cross-section. First adapter casing 204 includes a navigational channel 214a defined between proximal end 210a and distal end 212a on an inner surface 222a of first adapter casing 204.

First adapter casing 204 may include at least one first cooperating member 224 disposed on at least one engaging surface 226a of first adapter casing 204. In one exemplary configuration, the at least one first cooperating member 224 may be configured as a detent and have a thickened end 225. As depicted in FIG. 7, in one exemplary arrangement, the first adapter casing 204 has two first cooperating members 224 disposed adjacent to proximal end 210a of first adapter casing 204, and two first cooperating members 224 disposed adjacent to distal end 212a of first adapter casing 204, disposed on opposite sides of channel 214a. It should be noted, however, that the at least one first cooperating member 224 may be located anywhere on the at least one engaging surface 226a.

First adapter casing 204 also includes a mechanism for connecting mounting piece 208 to first adapter casing 204. In one exemplary arrangement (as depicted in FIG. 7), an engagement channel 228a disposed adjacent to distal end 212a. Engagement channel 228a extends inwardly from inner surface 222a of first adapter casing 204. As will be explained in further detail below, engagement channel 228a cooperates with a securing portion 234 of mounting piece 208 to secure mounting piece 208 to first and second adapter casings 204, 206.

With continued reference to FIG. 7, second adapter casing 206 extends partially about an axis E-E and is defined by a proximal end 210b and a distal end 212b. In one exemplary configuration, the second adapter casing 206 has a generally U-shaped cross-section. Second adapter casing 206 includes a navigational groove 214b defined between proximal end 210b and distal end 212a on an inner surface 222b of second adapter casing 206. In one exemplary arrangement, second adapter casing 206 is generally a mirror image of first adapter casing 204.

Second adapter casing 206 may include at least one second cooperating member 230 disposed on at least one engaging surface 226b of second adapter casing 206. The at least one second cooperating member 230 may be an indentation configured to permit engagement with a first cooperating member 224 in a complementary manner. In addition, an engagement tab 232 may be disposed within the indentations of second cooperating member 230 adjacent to an edge of the indentation of second cooperating member 230. With this arrangement, the thickened end of the first cooperating member 224 may snap-fit around the engagement tab 232 to assist in locking the first and second adapter casings 204 and 206 together. As depicted in FIG. 7, the second adapter casing 206 has two second cooperating members 230 disposed adjacent to proximal end 210b of second adapter casing 206, and two second cooperating members 230 disposed adjacent to distal end 212b of second adapter casing 206, disposed on either side of the navigation groove 214b, generally corresponding to the locations of the first cooperating members 224 of first adapter casing 204a. It should be noted, however, that the at least one second cooperating member 230 may be located anywhere on the at least one engaging surface 226b.

It should also be noted that, though FIG. 7 depicts first adapter casing 204 including four first cooperating members 224, and second adapter casing 206 including four second cooperating members 230, first adapter casing 204 may have any number of first cooperating members 224 greater than one, and second adapter casing 206 may have any number of second cooperating members 230 equal to or greater than the number of first cooperating members 224 that first adapter casing 204 has. Furthermore, first adapter casing 204 may have at least one second cooperating member 230 in addition to the at least one first cooperating member 224. Similarly, second adapter casing 206 may have at least one first cooperating member 224 in addition to the at least one second cooperating member 230. It should be noted that other engagement mechanisms may be used to secure first adapter casing 204 to second adapter casing 206.

Second adapter casing 206 also includes a mechanism for connecting mounting piece 208 to second adapter casing 204. According to the invention (as depicted in FIG. 7), second adapter casing 204 includes engagement channel 228b disposed adjacent to distal end 212b. Engagement channel 228b extends inwardly from inner surface 222b of second adapter casing 206. As with engagement channel 228a, engagement channel 228b cooperates with securing portion 234 of mounting piece 208 to secure mounting piece 208 to first and second adapter casings 204, 206. It should be noted that other engagement mechanisms may be used to secure mounting piece 208 to first and second adapter casings 204, 206.

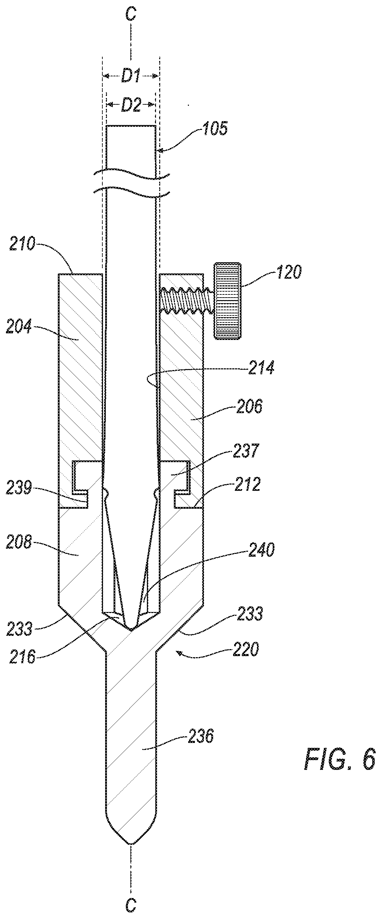

With continued reference to FIG. 7, mounting piece 208 is defined by a proximal end 218 and a distal end 220, and extends about an axis E-E. Distal end 220 of mounting piece 208 includes an annular angled surface 233 (best seen in FIG. 6). Angled surface 233 tapers from an outside diameter of the mounting piece 208 toward a positioning member 236 that extends from the distal end 220. Similar to adapter 102, angled surface 233 is configured to be complementary to chamfer 125 to seat the mounting piece 208 within obturator 104.

Mounted to a proximal face 235 of the proximal end 218 of mounting piece 208 is the securing portion 234. Securing portion 234 is configured to engage with engagement channels 228a, 228b of first and second adapter casings 204, 206 to form assembled adapter 202 (FIG. 5). Referring to FIG. 6, securing portion 234 is defined by a flange member 237 that is spaced from the proximal face 235 such that an annular groove 239 is formed between the flange member 237 and proximal face 235. The flange member 237 has a diameter that is smaller than a diameter of the mounting piece 208.

A stopper portion 244 may extend outwardly from the flange member 237. Stopper portion 244 may be received in a complementary key hole (not shown) formed in either of first and second adapter casings 204, 206, within engagement channels 228a, 228b, respectively, to prevent rotation of mounting piece 208 with respect to assembled first and second adapter casings 204, 206.

A mounting opening 238 is formed through the securing portion 234. Mounting opening 238 is in communication with a mounting piece channel 240. Mounting piece channel 240 may be constructed of a predetermined size to accommodate navigational element 105 (FIG. 5) therein. Mounting piece channel 240 terminates in a closed distal end. Closed distal end may be configured to taper inwardly to define a seating portion 216 that receives the distal tip 103 of navigational element 105, similar to the seating portion 126 described above in connection with adapter 102. More specifically, similar to seating portion 126, seating portion 216 may be annularly angled such that when navigational element 105 contacts seating portion 216, navigational element 105 is properly seated within adapter 202 so as to be aligned with a top surface of obturator 104.

To assemble adapter 202, the flange member 237 is inserted within engagement channel 228a, 228b, with the stopper element positioned within a key hole. Simultaneously, the first and second casings 204, 206 are brought into engagement with one another such that at least one first cooperating member 224 of first adapter casing 204 engages with the at least one second cooperating member 230 of second adapter casing 206. This engagement may be a snap-fit arrangement that locks first and second adapter casings 204, 206 together in a cooperating manner, thereby trapping the flange member 237 within the first and second casings 204, 206. When first adapter casing 204 is assembled with second adapter casing 206, navigational channels 214a, 214b combine to form the navigational channel 214 that is in communication with mounting piece channel 240 (FIG. 6) to form a continuous channel. The continuous channel is in communication with proximal opening 127 of obturator, when adapter 202 is assembled to obturator 104.

Once assembled, navigation element 105 may be disposed within combined navigational channel 214/mounting piece channel 240, such that the distal tip 103 is directed into seating portion 216. Once positioned, locking mechanism 120 may be actuated to lock the navigational element 105 within adapter 202.

In some implementations, a window 203a may be disposed on at least one of the first and second adapter casings 204, 206. Window 203a may extend partially about an axis C-C between proximal end 210 and distal end 212 of assembled first and second adapter casings 204, 206. Alternatively or additionally, a window 203b may be disposed on mounting piece 208, enabling a user to visually confirm that navigational element 105 is in proper contact with seating portion 216 (FIG. 5). In some implementations, Window 203b may be offset by 90 degrees from window 203a. Similar to window 128, windows 203 a, 203b may allow a user to visually confirm the placement of navigational element 105 when inside adapter 202. Furthermore, windows 203a, 203b serve to reduce the weight of adapter 202, thereby making adapter 202 easier to manipulate in the surgical field. Reducing the weight of adapter 202 also reduces physician fatigue during a surgical procedure. Adapter 202 having both windows 203a, 203b may be advantageous in allowing a user to ensure proper placement of navigational element 105 by viewing navigational element 105 from two different angles. In some implementations, at least a portion of at least one of first adapter casing 204, second adapter casing 206, and mounting piece 208 may be made of a transparent material, also allowing a user to visually confirm the placement of navigational element 105 when inside adapter 202.

Once assembled, navigational channel 214 may extend along axis C-C and define a first diameter DI. Navigational element 105 may also have a second diameter D2, which is less than DI and allows navigational element 105 to be slidingly received within navigational channel 214.

When navigational element 105 is positioned within adapter 202, navigational element 105 will extend beyond proximal end 210 of first and second adapter casings 204, 206 in a direction away from adapter 202. This allows navigational element 105 to be connected to a navigational system (not shown) while navigational element 105 is positioned within adapter 202. Furthermore, when assembled with adapter 202, navigational element 105 extends beyond distal end proximal end 210 of first and second adapter casings 204, 206 and within mounting piece 208, allowing navigational element 105 to engage seating portion 216.

Once navigational element 105 is secured to the actuator 202, the adapter 202 is joined to the obturator 104. More specifically, the positioning member 236 is inserted within the proximal opening 127 of the obturator 104. As the positioning member 236 is elongated, a portion of the positioning member 236 will be disposed distally within the obturator 104, spaced from the top surface of the obturator 104, thereby providing stability of the engagement, as shown in FIG. 5. The annular angled surface 233 of the mounting piece 208 engages the annular chamfer 125 of the obturator 104 in a complementary manner. Once positioned, a locking mechanism 150 may be actuated to lock the positioning member 236 to the obturator 104, as described above. Further, once so assembled, the seating portion 216 serves to position the distal tip 103 of the navigational element 105 in alignment with the top surface 129 of the obturator 104. The proximal end 107 of the navigational element 105 is secured to the navigational system. Thus, when the obturator 104 is assembled to the outer sheath 104 of a surgical access assembly, because the offset X between the top surface 129 and the distal tip 115 of the obturator 104 is a predefined length, the location of the distal tip 115 may be determined while the surgical access assembly is in use, as the distal tip 103 of the navigational element 105 will be known by correlating the offset X with the location of the distal tip 103 of the navigational element 105.

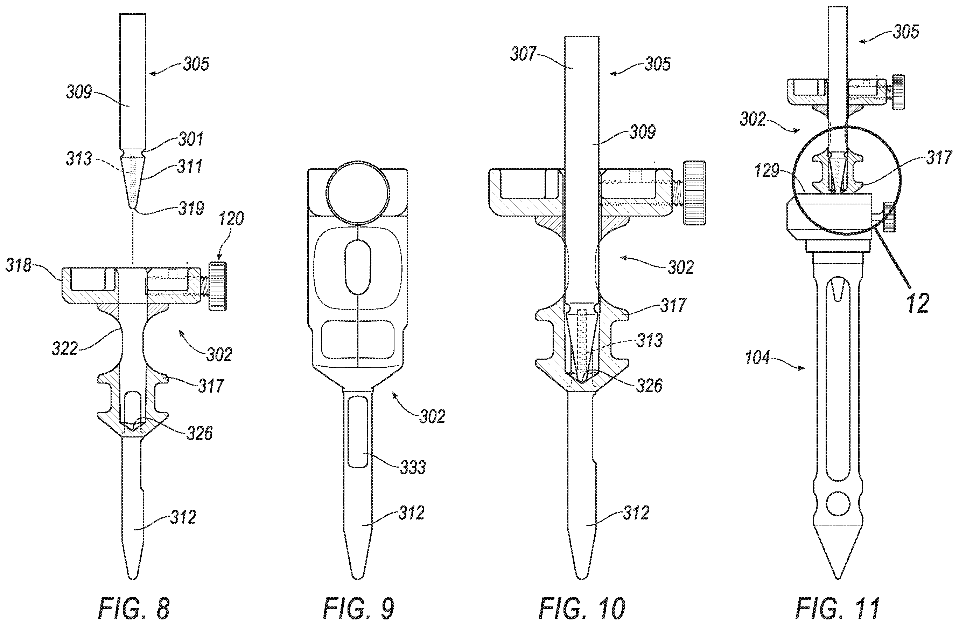

Referring to FIGS. 8-12, a further alternative arrangement of an adapter 302 will be described. Adapter 302 generally includes similar elements as described in connection with adapter 102, including handle 318, adapter body 317, positioning member 312 and seating portion 326. Like elements from the description of adapter 102 have been given similar reference numbers, increased by 200. Seating portion 326 is positioned to align with a top surface 129 of obturator 104 such that when a distal tip 319 is nested within seating portion 326, distal tip 319 is aligned with top surface 129 of obturator 104, as described above.

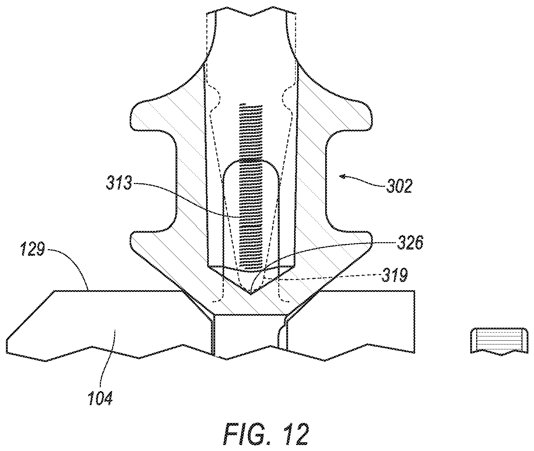

Much like that described above in connection with adapter 102, the adapter 302 is configured to selectively receive the navigational element 305. The navigational element 305 is defined by a proximal end 307, a body portion 309 and a distal end 311. The distal end 311 may be configured to taper inwardly from the body portion 309, terminating in distal tip 319. Disposed within the distal end 311 is an electromagnetic coil 313. However, electromagnetic coil 313 is slightly spaced from a distal tip 319 of the navigational element 305. Thus, as may be seen best in FIG. 12, when navigational element 305 is seated within seating portion 326, electromagnetic coil 313 is disposed slightly above the top surface 129 of obturator 104. With this arrangement, any interference between the obturator material and signals from the electromagnetic coil 313 of the navigational element 305 may be avoided.

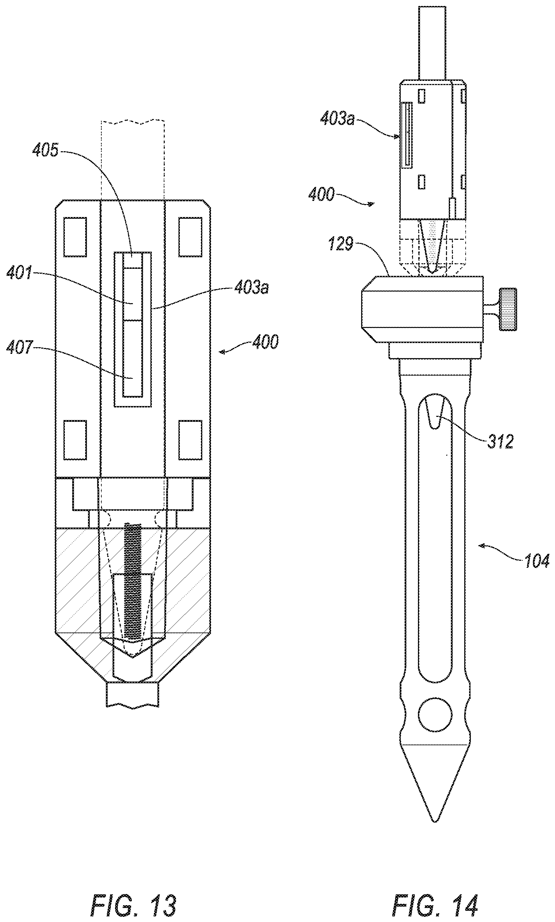

Referring to FIGS. 13-14, a further alternative arrangement of an adapter 400 is shown. Adapter 400 is similar to adapter 202. Like elements from the description of adapter 202 have been given similar reference numbers, increased by 200. While adapter 202 provides for navigational element 105/305 to be retained within the channel 214 via the cooperating sizes of the channel 214 and an outside diameter of the navigational element 105/305 and/or use of a locking mechanism 120, as a further alternative, an inside surface of the channel portions 214a, 214b may be formed with a compressible material to further frictionally retain the navigational element 105, thereby relaxing manufacturing tolerances between the channel 214 and the navigational element 105.

As yet a further exemplary arrangement, windows 403a may be provided with a spring element 401. Spring element 401 includes a first end 405 and a free end 407. The first end 405 is fixed to a proximal end of the window 403a, with the free end 405 biased to encroach within the channel 414. When navigational element 105/305 is disposed within the channel 414, the free end 405 of each spring element 401 will frictionally engage the outside surface of the navigational element 105/305, thereby retaining the navigational element 105/305 within the channel 414.

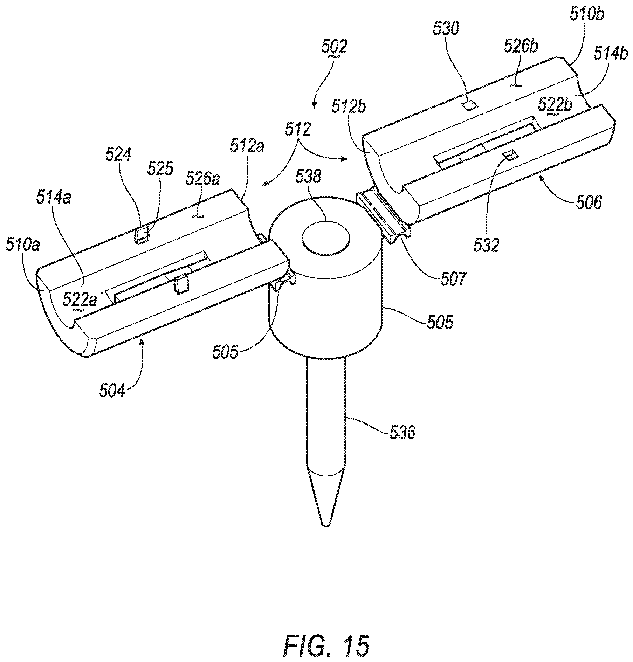

Referring to FIG. 15, a further embodiment of a navigation adapter 502 for use with an obturator of a surgical access system. Adapter 502 is similar to adapters 200 and 400. Like elements from the description of adapter 200 have been given similar reference numbers, increased by 300. Adapter 502 is defined by first and second adapter casings 504, 506, a mounting piece 508 from which a positioning member 536 extends.

The first and second adapter casings 504, 506 are each defined by a proximal end 510 and a distal end 512. In some implementations, at least one of first adapter casing 504, second adapter casing 506, and mounting piece 508 may include locking mechanism 120 (best seen in FIG. 5) or may include a spring element 401.

First adapter casing 504 has a generally U-shaped cross-section and includes a navigational groove 514a defined between proximal end 510a and distal end 512a on an inner surface 522a of first adapter casing 504. First adapter casing 504 may include at least one first cooperating member 524 disposed on at least one engaging surface 526a of first adapter casing 504. In one exemplary configuration, the at least one first cooperating member 524 may be configured as a detent and have a thickened end 525. As depicted in FIG. 15, in one exemplary arrangement, the first adapter casing 504 has two first cooperating members 524 disposed generally at the center of the first adapter casing 504, on either side of the navigation groove 514a. However, it is understood that this disclosure is not limited to this arrangement. Moreover, it is understood that additional cooperating members may be provided, similar to the arrangement depicted with respect to first adapter casing 204. First adapter casing 504 is hingedly connected to mounting piece 508 to first adapter casing 204 by hinge element 505.

Second adapter casing 506 is generally a mirror image of first adapter casing 504 and has a generally U-shaped cross-section and includes a navigational groove 514b defined between proximal end 510b and distal end 512b on an inner surface 522b of second adapter casing 506. Second adapter casing 506 may include at least one second cooperating member 530 disposed on at least one engaging surface 526b of second adapter casing 506.

The at least one second cooperating member 530 may be an indentation configured to permit engagement with a first cooperating member 524 in a complementary manner. In addition, an engagement tab 532 may be disposed within the indentations of second cooperating member 530 adjacent to an edge of the indentation of second cooperating member 530. With this arrangement, the thickened end of the first cooperating member 524 may snap-fit around the engagement tab 532 to assist in locking the first and second adapter casings 504 and 506 together.

As depicted in FIG. 15, in one exemplary arrangement, the second adapter casing 506 has two second cooperating members 530 disposed generally at the center of the second adapter casing 506, on either side of the navigation groove 514b so as to generally correspond with the locations of the first cooperating members 524. However, it is understood that this disclosure is not limited to this arrangement. Moreover, it is understood that additional cooperating members may be provided, similar to the arrangement depicted with respect to second adapter casing 506. Second adapter casing 506 is hingedly connected to mounting piece 508 by hinge element 507.

Mounting piece 508 is defined by a proximal end 518 and a distal end 520. Distal end 520 of mounting piece 508 includes an annular angled surface (similar to that shown in FIG. 6). The angled surface tapers from an outside diameter of the mounting piece 508 toward a positioning member 536 that extends from the distal end 520. Similar to adapter 102, the angled surface is configured to be complementary to chamfer 125 to seat the mounting piece 508 within obturator 104.

A mounting opening 538 is formed through the proximal end 518. Mounting opening 538 is in communication with a mounting piece channel, similar to mounting piece channel 240. The mounting piece channel may be constructed of a predetermined size to accommodate navigational element 105 (FIG. 5) therein. The mounting piece channel terminates in a closed distal end and may be configured to taper inwardly to define a seating portion similar to seating portion 216 that receives the distal tip 103 of navigational element 105.

To assemble adapter 502, the first and second casings 204, 206 are pivoted from a non-operational position (as shown in FIG. 15) about the hinge elements 505 and 507 brought into engagement with one another such that at least one first cooperating member 524 of first adapter casing 504 engages with the at least one second cooperating member 530 of second adapter casing 506. This engagement may be a snap-fit arrangement that locks first and second adapter casings 504, 506 together in a cooperating manner. When first adapter casing 504 is assembled with second adapter casing 506, navigational grooves 514a, 514b combine to form the navigational channel 514 that is in communication with the mounting piece channel, similar to that shown in FIG. 6 to form a continuous channel. The continuous channel is in communication with proximal opening 127 of obturator, when adapter 502 is assembled to obturator 104.

Once assembled, navigation element 105 may be disposed within combined navigational channel 514/mounting piece channel, such that the distal tip 103 is directed into the seating portion. Once positioned, a locking mechanism 120 may be actuated to lock the navigational element 105 within adapter 502, if provided.

It will be appreciated that the surgical access system described herein has broad applications. The foregoing embodiments were chosen and described in order to illustrate principles of the apparatuses as well as some practical applications. The preceding description enables others skilled in the art to utilize apparatuses in various embodiments and with various modifications as are suited to the particular use contemplated. In accordance with the provisions of the patent statutes, the principles and modes of operation of this disclosure have been explained and illustrated in exemplary embodiments.

It is intended that the scope of the present apparatuses be defined by the following claims. However, it must be understood that this disclosure may be practiced otherwise than is specifically explained and illustrated without departing from the claims. It should be understood by those skilled in the art that various alternatives to the embodiments described herein may be employed in practicing the claims without departing from the scope as defined in the following claims. The scope of the disclosure should be determined, not with reference to the above description, but should instead be determined with reference to the appended claims. It is anticipated and intended that future developments will occur in the arts discussed herein, and that the disclosed systems will be incorporated into such future examples. Furthermore, all terms used in the claims are intended to be given their broadest reasonable constructions and their ordinary meanings as understood by those skilled in the art unless an explicit indication to the contrary is made herein. In particular, use of the singular articles such as "a," "the," "said," etc. should be read to recite one or more of the indicated elements unless a claim recites an explicit limitation to the contrary. It is intended that the following claims define the scope of the invention and that the apparatus within the scope of these claims be covered thereby. In sum, it should be understood that the invention is capable of modification and variation and is limited only by the following claims.

## Claims

1. An adapter (202) for a surgical access assembly, comprising:
a first adapter casing (204);
a second adapter casing (206) selectively engageable with the first adapter casing (204); and
a mounting piece (208) selectively engaged with the first and second adapter casings (204, 206), wherein in an engaged position the mounting piece (208) is positioned distally relative to the first and second adapter casings (204, 206);
wherein the first adapter casing (204) and second adapter casing (206) cooperate to define a channel (214) that is configured to receive a navigation element (105), wherein the mounting piece (208) defines a mounting piece channel (240) that terminates in a closed distal end configured to receive a distal tip (103) of the navigation element (105), wherein the channel (214) is in communication with the mounting piece channel (240) to form a continuous channel in the engaged position,
wherein a portion of the mounting piece (208) is disposed within a portion of an inner surface of the first adapter casing (204) and a portion of an inner surface of the second adapter casing (206) to secure the mounting piece (208) to the first and second adapter casings (204, 206); and
wherein the first adapter casing (204) includes an engagement channel (228a) adjacent to the distal end of the first adapter casing (204), the second adapter casing (206) includes an engagement channel (228b) adjacent to the distal end of the second adapter casing (206), and the mounting piece (208) includes a securing portion (234) that engages with the engagement channel (228a) of the first adapter casing (204) and the engagement channel (228b) of the second adapter casing (206).

2. The adapter (202) of claim 1, wherein the first adapter casing (204) includes a first navigation groove (214a) defined between a proximal end of the first adapter casing (204) and a distal end of the first adapter casing (204), and wherein the second adapter casing (206) includes a second navigation groove (214b) defined between a proximal end of the second adapter casing (206) and a distal end of the second adapter casing (206), the first and second navigation grooves collectively defining the channel (214).

3. The adapter (202) of claim 1, wherein an engagement surface of the first adapter casing (204) defines one or more first cooperating members (224), and wherein an engagement surface of the second adapter casing (206) includes one or more second cooperating members (230) configured to engage with the one or more first cooperating members (224) of the inner surface of the first adapter casing (204) to connect the first and second adapter casings (204, 206) together.

4. The adapter (202) of claim 1, wherein the mounting piece (208) further includes a window (203).

## Patentansprüche

1. Adapter (202) für eine chirurgische Zugangsvorrichtung, umfassend:
ein erstes Adaptergehäuse (204);
ein zweites Adaptergehäuse (206), das wahlweise mit dem ersten Adaptergehäuse (204) in Eingriff bringbar ist; und
ein Montagestück (208), das wahlweise mit dem ersten und dem zweiten Adaptergehäuse (204, 206) in Eingriff steht, wobei das Montagestück (208) in einer Eingriffsposition distal relativ zu dem ersten und dem zweiten Adaptergehäuse (204, 206) positioniert ist;
wobei das erste Adaptergehäuse (204) und das zweite Adaptergehäuse (206) zusammenwirken, um einen Kanal (214) zu definieren, der zur Aufnahme eines Navigationselements (105) eingerichtet ist, wobei das Montagestück (208) einen Montagestückkanal (240) definiert, der in einem geschlossenen distalen Ende endet, das zur Aufnahme einer distalen Spitze (103) des Navigationselements (105) eingerichtet ist, wobei der Kanal (214) mit dem Montagestückkanal (240) in Verbindung steht, um in der Eingriffsposition einen durchgehenden Kanal zu bilden,
wobei ein Abschnitt des Montagestücks (208) innerhalb eines Abschnitts einer Innenfläche des ersten Adaptergehäuses (204) und eines Abschnitts einer Innenfläche des zweiten Adaptergehäuses (206) angeordnet ist, um das Montagestück (208) an dem ersten und dem zweiten Adaptergehäuse (204, 206) zu befestigen; und
wobei das erste Adaptergehäuse (204) einen Eingriffskanal (228a) neben dem distalen Ende des ersten Adaptergehäuses (204) umfasst, das zweite Adaptergehäuse (206) einen Eingriffskanal (228b) neben dem distalen Ende des zweiten Adaptergehäuses (206) umfasst und das Montagestück (208) einen Befestigungsabschnitt (234) umfasst, der mit dem Eingriffskanal (228a) des ersten Adaptergehäuses (204) und dem Eingriffskanal (228b) des zweiten Adaptergehäuses (206) in Eingriff steht.

2. Adapter (202) nach Anspruch 1, wobei das erste Adaptergehäuse (204) eine erste Navigationsnut (214a) umfasst, die zwischen einem proximalen Ende des ersten Adaptergehäuses (204) und einem distalen Ende des ersten Adaptergehäuses (204) definiert ist, und wobei das zweite Adaptergehäuse (206) eine zweite Navigationsnut (214b) umfasst, die zwischen einem proximalen Ende des zweiten Adaptergehäuses (206) und einem distalen Ende des zweiten Adaptergehäuses (206) definiert ist, wobei die erste und die zweite Navigationsnut gemeinsam den Kanal (214) definieren.

3. Adapter (202) nach Anspruch 1, wobei eine Eingriffsfläche des ersten Adaptergehäuses (204) ein oder mehrere erste zusammenwirkende Elemente (224) definiert und wobei eine Eingriffsfläche des zweiten Adaptergehäuses (206) ein oder mehrere zweite zusammenwirkende Elemente (230) umfasst, die so eingerichtet sind, dass sie mit dem einen oder den mehreren ersten zusammenwirkenden Elementen (224) der Innenfläche des ersten Adaptergehäuses (204) in Eingriff stehen, um das erste und das zweite Adaptergehäuse (204, 206) miteinander zu verbinden.

4. Adapter (202) nach Anspruch 1, wobei das Montagestück (208) weiterhin ein Fenster (203) umfasst.

## Revendications

1. Adaptateur (202) pour un ensemble d'accès chirurgical, comprenant:
un premier boîtier d'adaptateur (204);
un second boîtier d'adaptateur (206) pouvant être sélectivement inséré dans le premier boîtier d'adaptateur (204); et
un élément de fixation (208) sélectivement introduit dans les premier et second boîtiers d'adaptateur (204, 206), dans lequel, lorsqu'il est introduit, l'élément de fixation (208) est positionné distalement par rapport aux premier et deuxième boîtiers d'adaptateur (204, 206);
dans lequel le premier boîtier adaptateur (204) et le second boîtier adaptateur (206) coopèrent pour définir un canal (214) conçu pour recevoir un élément de navigation (105), dans lequel l'élément de fixation (208) définit un canal d'élément de fixation (240) qui se termine par une extrémité distale fermée conçue pour recevoir une pointe distale (103) de l'élément de navigation (105), dans lequel le canal (214) est en communication avec le canal d'élément de fixation (240) pour former un canal continu lorsque l'élément de fixation est introduit,
dans lequel une partie de l'élément de fixation (208) est disposée à l'intérieur d'une partie de la surface intérieure du premier boîtier d'adaptateur (204) et d'une partie de la surface intérieure du second boîtier d'adaptateur (206) afin de fixer l'élément de fixation (208) aux premier et second boîtiers d'adaptateur (204, 206); et
dans lequel le premier boîtier d'adaptateur (204) comprend un canal d'engagement (228a) adjacent à l'extrémité distale du premier boîtier d'adaptateur (204), le second boîtier d'adaptateur (206) comprend un canal d'engagement (228b) adjacent à l'extrémité distale du second boîtier d'adaptateur (206), et la élément de fixation (208) comprend une partie de fixation (234) qui s'engage dans le canal d'insertion (228a) du premier boîtier d'adaptateur (204) et dans le canal d'insertion (228b) du second boîtier d'adaptateur (206).

2. Adaptateur (202) selon la revendication 1, dans lequel le premier boîtier d'adaptateur (204) comprend une première rainure de navigation (214a) définie entre une extrémité proximale du premier boîtier d'adaptateur (204) et une extrémité distale du premier boîtier d'adaptateur (204), et dans lequel le second boîtier d'adaptateur (206) comprend une seconde rainure de navigation (214b) définie entre une extrémité proximale du second boîtier d'adaptateur (206) et une extrémité distale du second boîtier d'adaptateur (206), les première et seconde rainures de navigation définissant collectivement le canal (214).

3. Adaptateur (202) selon la revendication 1, dans lequel une surface d'insertion du premier boîtier d'adaptateur (204) définit un ou plusieurs premiers éléments de coopération (224), et dans lequel une surface d'insertion du second boîtier d'adaptateur (206) comprend un ou plusieurs seconds éléments de coopération (230) conçus pour entrer en prise avec un ou plusieurs premiers éléments de coopération (224) de la surface intérieure du premier boîtier d'adaptateur (204) pour assembler conjointement le premier et le second boîtiers d'adaptateur (204, 206).

4. Adaptateur (202) selon la revendication 1, dans lequel l'élément de fixation (208) comprend en outre une fenêtre (203).
